(19) Europäisches Patentamt
European Patent Office
Office européen des brevets

(11) Publication number: **0 414 299 B1**

(12) **EUROPEAN PATENT SPECIFICATION**

(45) Date of publication of patent specification: **15.06.94**

(51) Int. Cl.[5]: **C07C 281/20**, C07C 259/20, C07C 291/02, A01N 37/52

(21) Application number: **90202123.7**

(22) Date of filing: **03.08.90**

(54) **Biocidal azoxime compounds.**

(30) Priority: **04.08.89 GB 8917854**

(43) Date of publication of application:
**27.02.91 Bulletin 91/09**

(45) Publication of the grant of the patent:
**15.06.94 Bulletin 94/24**

(84) Designated Contracting States:
**CH DE ES FR GB GR IT LI NL**

(56) References cited:
**US-A- 4 270 947**

**CHEMICAL ABSTRACTS, vol. 83, 1975, page 141, abstract no. 127289v, Columbus, Ohio, US; E.P. NESYNOV et al.: "Aryl esters and amides of substiuted carboxylic, carbazinic, and thioimido acids and O-aryl oximes and their effectiveness in controlling plant diseases", & FIZIOL. AKT. VESHCHESTVA 1975, 7, 49-53**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Gray, Andrew Clement Gripper**
**63 New Road**
**London E1 1HH(GB)**
Inventor: **Wood, William Wakefield**
**37 Hawrhorne Road**
**Sittingbourne, Kent(GB)**
Inventor: **Naisby, Thomas Webster**
**79 Gadby Road**
**Sittingbourne, Kent(GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 86, 1977, page 471, abstract no. 88976g, Columbus, Ohio, US; A.S. SHAWALI et al.: "Substituent effects on the ionization constants of phenylazobenzaldoximes", & J. CHEM. 1976, 54(20), 3260-6

CHEMICAL ABSTRACTS, vol. 87, 1977, page 434, abstract no. 38406w, Columbus, Ohio, US; K.C. KALIA et al.: "Methylation of arylazo oximes with dimethyl sulfate", & INDIAN J. CHEM., SECT. B 1976, 14B(11), 826-8

CHEMICAL ABSTRACTS, vol. 88, 1978, page 472, abstract no. 57743r, Columbus, Ohio, US; I.M. ABBASS: "Pyridine nitrosazones and their cobalt(III) chelates", & CAN. J. CHEM. 1977, 55(21), 3707-11

CHEMICAL ABSTRACTS, vol. 100, 1984, page 685, abstract no. 113933h, Columbus, Ohio, US; C. NATARAJAN et al.: "Iron(II), cobalt(III), nickel(II) and copper(II) chelates of furan and thiophene azo-oximes", & TRANSITION MET. CHEM. (WEINHEIM, GER.) 1984, 9(1), 18-22

JOURNAL OF ORGANIC CHEMISTRY, vol. 35, no. 7, 1970, pages 2231-2235; K.C. KALIA et al.: "Hydrogen bonding and isomerism in arylazo oximes"

CHEM. BER., vol. 115, 1982, pages 3706-3718, Verlag Chemie GmbH, Weinheim, DE; F.A. NEUGEBAUER et al.: "Arylazo(R)methaniminoxyle, Beispiel einer "N.O-Dreielektronen-delta-Bindung"?"

JOURNAL OF THE CHEMICAL SOCIETY, PERKIN I, 1974, pages 81-85; J.A. MADDISON et al.: "Some trans-phenylazo-1,2,4-oxadiazoles"

## Description

This invention relates to the use of certain phenyl azoxime compounds as biocides, especially fungicides, to biocidal compositions containing such compounds, to certain novel compounds of this type, and to the preparation of such novel compounds.

The N-oxides of certain compounds of the present invention form the subject of copending application no. EP-A-411717.

Japanese Patent Application No. 45-7079 discloses certain phenylformaldoxime compounds as being fungicidal.

U.S patent No. 4425271 discloses certain (phenylazo)-2,6-dichlorobenzaldimino esters as being herbicidal. Herbicidal (phenylazo)-2,6-dichlorobenzaldoxime precursors are also described.

Can. J.Chem. (1976), 54(20), pp. 3260-6 discloses twelve phenylazobenzaldoximes of the formula

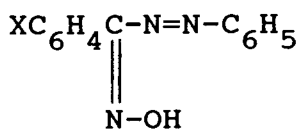

in which X is 4-OMe, 4-Me, H, 4-Cl, 4-Br, 3-Cl, 3-Br, 3-$NO_2$, 4-$NO_2$, 3,4-$O_2CH_2$, 2-Cl or 2-$NO_2$.

Indian J.Chem., Sect.B, (1976), 14B(11), pp. 826-8 discloses two arylazooximes of the formula

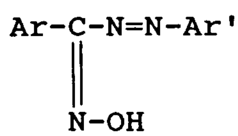

in which Ar represents a phenyl or 4-methylphenyl group and Ar' represents a phenyl group.

Can. J.Chem., (1977), 55(21), pp. 3707-11 discloses four azooximes of the formula

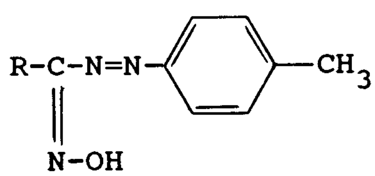

where R represents a 2-,3- or 4-pyridyl group or a phenyl group.

Transition Met.Chem., (1984), 9(1), pp 18-22 discloses four arylazooximes of the formula

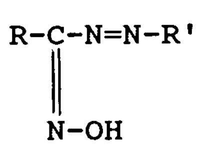

where R represents a 2-furyl or 2-thienyl group and R' represents a phenyl or para-tolyl group.

J.Org.Chem., (1970), 35(7), pp. 2231-4 discloses various arylazooximes of the formula

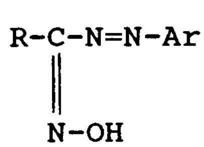

in which Ar represents a phenyl group and R represents a methyl, n-propyl, phenyl, para-tolyl or benzyl group or in which Ar represents a para-tolyl group and R represents a phenyl, para-tolyl, 1-naphthyl or 9-anthryl group.

Chem.Ber., (1982), 115, pp. 3706-3718 discloses various arylazoaldehyde oximes of the formula

$R^1$-N = N-C$R^3$ = N-OH

in which $R^1$ represents a phenyl group and $R^3$ represents a methyl, trifluoromethyl, benzyl, phenyl, 4-nitrophenyl or 4-methoxyphenyl group or in which $R^1$ represents a 4-nitrophenyl group and $R^3$ is a phenyl group.

J.Chem.Soc.Perkin I, (1974), pp. 81-85 explicitly discloses trans-phenylazoformamide oximes of the formula

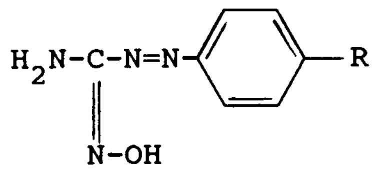

in which R is H, Cl, Br, OMe, $NO_2$ or Me.

The present invention is based upon the discovery of the activity of certain phenylaldoxime compounds, and derivatives thereof, many of which are new, in combating fungi, including plant pathogenic fungi. The compounds are characterised by substitution of the carbon backbone atom.

According to a first aspect of the present invention there is provided a method of combating a fungus at a locus, which comprises treating the locus with a compound of the general formula

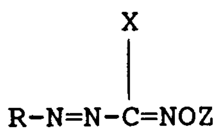

(I)

where R represents an optionally substituted aryl or heteroaryl group; X represents an amino group or an optionally substituted alkyl, aryl or heteroaryl group; and Z represents a hydrogen atom or a group of formula -C$OR^1$ where $R^1$ represents an optionally substituted alkyl group.

Unless otherwise specified in this specification, an alkyl group may be linear or branched and suitably contains up to 10, preferably up to 6, and most preferably up to 4, carbon atoms, preferred examples being methyl and ethyl.

A preferred optionally substituted aryl group is optionally substituted phenyl.

A heteroaryl group may be monocyclic or multicyclic. It may suitably contain 5 to 10 ring atoms, hetero atoms suitably being selected from nitrogen, oxygen and sulphur atoms, 1 to 4 ring atoms suitably being hetero atoms.

Unless otherwise stated in this specification, when any groups are designated as being optionally substituted, the substituent groups which are optionally present may be any of those customarily employed in the development of biocidal compounds, and/or the modification of such compounds to influence their structure/activity, persistence, penetration or other property. In relation to alkyl groups, specific examples of such substituents include halogen, especially fluorine, chlorine or bromine atoms, and phenyl, cyano, amino, hydroxy, alkoxy, (alkyl)amino and haloalkyl groups. In relation to an aryl or heteroaryl moiety, optional substituents include halogen atoms, for example fluorine, chlorine, bromine and iodine atoms, and nitro, cyano, amino, (alkyl)amino, hydroxy, alkoxy, alkyl and haloalkyl (e.g. $CF_3$) groups.

Preferably, R represents an optionally substituted phenyl group, especially a phenyl or halophenyl, preferably chlorophenyl, group. Particularly preferred is a halophenyl group having halogen at the 4-position, and being optionally substituted elsewhere in the ring.

Preferably, X represents an optionally substituted, preferably unsubstituted, alkyl or phenyl group, an amino group, or a thienyl or pyridyl group (optionally an N-oxide thereof).

It should be noted that compounds of the general formula I may exist as cis or trans isomers, and that the scope of the present invention covers any such isomers, isolated or together.

In the method of the invention as described above the locus may be an agricultural or horticultural locus, for example plants subject to attack, seeds of such plants or the medium in which such plants are

growing or are to be grown. Compounds of the present invention have been shown to exhibit activity against a range of important fungi, including vine downy mildew, vine grey mould, wheat leafspot, barley powdery mildew, tomato early blight, wheat eyespot, seedling wheat blight and wheat brown rust. Such a locus as mentioned above may suitably be treated with a compound I at an application rate in the range 0.05-4 kg/ha, preferably 0.1-1 kg/ha.

The invention further provides the use as a fungicide of a compound of the general formula I as defined in any of the above statements.

Nematicidal activity has also been found in compounds of general formula I and nematicidal use of the compounds constitutes a further aspect of the invention.

According to a further aspect of the invention there is provided a compound of the general formula I, as defined in any of the foregoing statements, per se, with the provisos that

(i) X does not represent an 2,6-dichlorophenyl group;

(ii) when Z represents a hydrogen atom and R represents a phenyl group, then X does not represent a phenyl,2-chlorophenyl, 3-chlorophenyl,4-chlorophenyl, 3-bromophenyl,4-bromophenyl,2-nitrophenyl, 3-nitrophenyl,4-nitrophenyl,4-methylphenyl, 4-methoxyphenyl,3,4-methylenedioxyphenyl,2-furyl,2-thienyl,methyl,n-propyl,benzyl or trifluoromethyl group;

(iii) when Z represents a hydrogen atom and R represents a 4-methylphenyl group, then X does not represent a phenyl,4-methylphenyl, 1-naphthyl,9-anthryl,2-furyl,2-thienyl,2-pyridyl,3-pyridyl or 4-pyridyl group;

(iv) when Z represents a hydrogen atom and R represents a 4-nitrophenyl group, then X does not represent a phenyl group; and

(v) when Z represents a hydrogen atom and X represents an amino group, then R does not represent a phenyl,4-chlorophenyl, 4-bromophenyl,4-nitrophenyl,4-methylphenyl or 4-methoxyphenyl group.

Further in accordance with the invention there is provided a fungicidal composition which comprises a carrier and, as active ingredient, a novel compound of general formula I, as defined above.

A carrier in a composition according to the invention is any material with which the active ingredient is formulated to facilitate application to the locus to be treated, or to facilitate storage, transport or handling. A carrier may be a solid or a liquid, including a material which is normally gaseous but which has been compressed to form a liquid, and any of the carriers normally used in formulating biocidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexenone; ethers; aromatic or araliphatic hydrocarbons, for example benzene, toluene and xylene; petroleum fractions, for example kerosene and light mineral oils; chlorinated hydrocarbons, for example carbon tetrachloride, perchloroethylene and trichloroethane. Mixtures of different liquids are often suitable.

Compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emusifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing 0.5-10% w of active ingredient. Granules are usually prepared to have a size between 10 and 100 BS mesh (1.676 - 0.152 mm), and may be manufactured by agglomeration or impregnation techniques. Generally, granules will contain 0.5-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick "mayonnaise" - like consistency.

The compositions of the invention may also contain other ingredients, for example other compounds possessing herbicidal, insecticidal or fungicidal properties.

In accordance with a further aspect of the invention there is provided a process for the preparation of a novel compound, as defined above, of the general formula I, which comprises treating a compound of the general formula

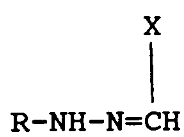 (II)

where R and X are as defined above but X is not an amino group, with a higher alkyl nitrite in the presence of a base; and optionally derivatising the product to produce a further novel compound of general formula I. The reaction with the nitrite suitably takes place in an organic solvent, such as an alcohol, at a temperature in the range 40-120 °C, preferably under reflux. Suitable bases include alkali metal alkoxides. Particularly suitable base/solvent systems include sodium ethoxide/ethanol and sodium methoxide/methanol. Acidification completes the step. The product is a compound in which Z is hydrogen.

Further information on this method is obtainable in Berichte XXXVI, 85, 1903 (Bamberger, E., and Pemsel, w.) and in U.S. Patent No. 4425271.

Novel compounds of general formula I wherein X represents an amino group may be prepared by treating a compound of general formula $R-N_2CN$ (suitably obtained by diazotisation of an aniline $R-NH_2$, followed by cyanuration) with hydroxylamine or an acid addition salt thereof, in the presence of a base, for example sodium hydroxide, to yield a compound of formula I in which X represents an amino group and Z represents a hydrogen atom; and optionally derivatising the product to produce a further novel compound of general formula I. Acidification precipitates the product.

Further information on the above method is contained in Gazz. Chem. Ital., 63, 923-926, 1933 (G. Long).

In accordance with a further aspect of the present invention there is provided a further process for the preparation of a novel compound of general formula I, where R and X are as defined above but X is not an amino group, which comprises treating a compound of the general formula

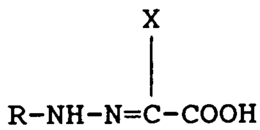 (III)

with a base and a nitrite salt, to yield a compound of general formula I wherein Z is hydrogen; and optionally derivatising that compound to produce a further novel compound of general formula I.

Suitably, the reaction takes place in the presence of a solvent, for example water. The reaction is preferably effected at a temperature in the range 40 °C to the reflux temperature. The base may be, for example, sodium carbonate. The nitrite may be an alkali metal salt, for example sodium nitrite. Acidification completes the step.

The compound of general formula III may be prepared by reaction of a hydrazine of general formula

$R\text{-}NH\text{-}NH_2$ (IV)

with a carboxylic acid of general formula X-C(O)-COOH (V). This reaction suitably takes place in a solvent, for example water or an organic solvent, at a temperature in the range -20 to 50 °C.

The reaction scheme described in the immediately preceding paragraphs is also described in Synth. Comm., 16(2), 163-167, 1968 (J.W. Lyga), and in J. Prakt. Chem., (2)75, 121, 1906, (M. Busch, E. Meussdorffer).

Derivatisation of a novel compound of formula I may be carried out, for example, to yield a compound of general formula I wherein Z is a group of formula $-COR^1$. To prepare a compound of general formula I wherein Z represents a group of general formula $-COR^1$, a compound of general formula I wherein Z represents a hydrogen atom is preferably reacted with an appropriate acid halide $R^1$-COHal, (preferably chloride) in the presence of a base, for example dimethylaniline, pyridine, sodium hydroxide or triethylamine, in an inert organic solvent, such as an ether or alcohol, at a temperature in the range 0-50 °C.

The starting materials described herein are known or else may be prepared from known compounds by standard methods.

The invention will now be further illustrated by the following examples.

Example 1

Preparation of 4-chlorophenylazobenzaldoxime
(R = 4-chlorophenyl; X = phenyl; Z = hydrogen; unoxidised).

Benzaldehyde phenylhydrazone (9.2 g; 0.04 mole) was added to sodium ethoxide (0.04 mole) in ethanol (100 ml). Amyl nitrite (12 ml; 0.09 mole) was added and the reaction mixture was refluxed for thirty minutes. The cooled reaction mixture was then poured into iced water (300 ml) containing 2M sodium hydroxide solution (40 ml). After washing with diethyl ether the aqueous layer was acidified with 2M sulphuric acid and extracted into diethyl ether. The solvent was removed and the product recrystallised from ethanol. Yield 5.4 g. mp 123 °C. m/e found 259/261.

| | | | | |
|---|---|---|---|---|
| Analysis: | Calc. % | C 60.1 | H 3.9 | N 16.2 |
| | Found % | C 60.1 | H 4.2 | N 16.3 |

Example 2

Preparation of amino-4-chlorophenylnitrosoazone
(R = 4-chlorophenyl; X = amino; Z = hydrogen; unoxidised).

p-Chloroaniline (25.5 g; 0.2 mole) was diazotised in 50% HCl (100 ml) with sodium nitrite (14 g; 0.2 mole) in water (80 ml). Aqueous potassium cyanide (13 g; 0.2 mole) in water (30 ml) was added dropwise at 0 °C then extracted into diethyl ether and the ice cold extract treated with hydroxylamine hydrochloride (14 g; 0.02 mole) in 2M sodium hydroxide solution (200 ml). The product was precipitated by dropwise addition of glacial acetic acid at 0 °C. Yield 0.7 g. mp 190-195 °C (decomp). m/e 198/200.

| Analysis: | Calc. % | C 42.3 | H 3.6 | N 28.2 |
|---|---|---|---|---|
| | Found % | C 42.2 | H 3.7 | N 27.4 |

Example 3

Preparation of phenylazoacetaldoxime
(R = phenyl; X = methyl; Z = hydrogen; unoxidised).

Acetaldehyde (30 g), in water (20 ml) and ethanol (120 ml), was treated with phenyl hydrazine (60 g), added in portions, with cooling. The reaction mixture was stirred at ambient temperature for about 2 hours until precipitation was complete. The phenylhydrazone product, $Ph-NH-N=CHCH_3$, was washed with a little aqueous ethanol, and dried. mp 95-97 °C. The phenylhydrazone (33 g) was added to sodium ethoxide (56 g sodium in 1 litre ethanol). Amyl nitrite (75 ml) was added and the reaction mixture was heated under reflux for about 1 hour. On cooling, the reaction mixture was poured into iced water (1.5 litre) containing 2M sodium hydroxide solution (250 ml). The solution was washed with diethyl ether to remove amyl nitrite, and the aqueous layer was acidified by addition of 2M hydrochloric acid, and extracted with diethyl ether. The diethyl ether was removed and the residue recrystallised from cyclohexane, yielding phenylazoacetaldoxime (19.6 g), mp. 118-120 °C. The mother liquors were flash chromatographed on silica, eluted with dichloromethane, to give a further 6.7 g.

| Analysis: | Calc. % | C 58.9 | H 5.5 | N 25.8 |
|---|---|---|---|---|
| | Found % | C 58.9 | H 5.5 | N 25.9 |

Further compounds were prepared according to procedures similar to those described in the preamble to the specification and in Examples 1, 2 and 3. Data on these compounds are set out in Table 1 below. In Table 1, reference is made to a compound of the following general formula : R-N = N-C(X) = NOZ

TABLE 1

| Compound of Example No. | R | X | Z | Anaylsis CHN Calc. % Found % | | | mp/bp (°C) | m/e |
|---|---|---|---|---|---|---|---|---|
| 4 | 3,4-$Cl_2$-Ph | Ph | H | 53.0<br>52.6 | 3.0<br>3.1 | 14.3<br>13.9 | 133-135 | 294 |
| 5 | 3,4-$Cl_2$-Ph | 3-pyridyl | H | 48.8<br>48.8 | 2.7<br>3.0 | 19.0<br>18.9 | 185-187 | 295 |
| 6 | 3,4-$Cl_2$-Ph | 3-pyridyl N-oxide | H | 46.3<br>45.6 | 2.6<br>2.8 | 18.0<br>17.3 | 204-206 | 310 |
| 7 | Ph | 2-thienyl | H | 57.1<br>56.8 | 3.9<br>3.9 | 18.2<br>18.0 | 99-100 | 231 |
| 8 | 4-F-Ph | 2-thienyl | H | 53.0<br>53.6 | 3.2<br>3.6 | 16.9<br>17.0 | 136-138 | 249 |

TABLE 1

| Compound of Example No. | R | X | Z | Anaylsis CHN Calc. % Found % | mp/bp (°C) | m/e |
|---|---|---|---|---|---|---|
| 9 | 4-Cl-Ph | $CH_3$ | H | 48.6 4.0 21.2<br>47.9 4.0 20.8 | 183-185 | 197/199 |
| 10 | Benzothiazol-2-yl | $CH_3$ | H | 49.1 3.7 25.5<br>49.0 3.7 25.5 | 203-205 | 220 |
| 11 | Ph | $CH_3$ | $COCH_3$ | 58.5 5.4 20.5<br>58.5 5.4 20.3 | 99-101 | 205 |
| 12 | Ph | $CH_3$ | $COCH_3$ | 54.3 5.0 19.0<br>54.5 5.1 19.1 | 66-68 | 221 |

Example 13

Preparation of phenylazoacetaldoxime-N-oxide
(R = phenyl; X = methyl; Z = hydrogen; N-oxide).

The compound of Example 3, phenylazoacetaldoxime (8.0 g; 0.05 mole) in $CH_2Cl_2$ (c. 100 ml) at -10 °C was treated with peroxytrifluoroacetic acid (100 ml of 0.5M solution in $CH_2Cl_2$) for about 1 hour. The reaction mixture was washed with dilute sodium bicarbonate solution (c. 100 ml) and dried over $MgSO_4$. The solvent was removed under vacuum and the brown solid recrystallised from cyclohexane. Yield 4.1 g. mp 121 °C. m/e 179.

| | | | | |
|---|---|---|---|---|
| Analysis: | Calc. %<br>Found % | C 53.7<br>C 53.7 | H 5.0<br>H 5.1 | N 23.4<br>N 23.3 |

Further compounds were prepared according to procedures similar to those described in the preamble and in Examples 1, 2, 3 and 13. Data on these compounds are set out in Table 2 below. In Table 2, reference is made to an N-oxide compound of the general formula I.

TABLE 2 (compounds of Formula I)

| Compound of Example No. | R | X | Z | Anaylsis CHN Calc. % Found % | mp/bp (°C) | m/e |
|---|---|---|---|---|---|---|
| 14 | 4-Cl-Ph | Ph | H | 56.6 3.7 15.3<br>55.8 3.7 15.0 | 142-144 | 275/277 |
| 15 | 4-Cl-Ph | $CH_3$ | H | 45.0 3.8 19.6<br>45.3 3.9 19.5 | 150-153 | 213/215 |
| 16 | 4-F-Ph | 2-thienyl | H | 49.8 3.0 15.8<br>49.8 3.1 15.7 | 175-177 | 265 |
| 17 | Ph | 2-thienyl | H | 53.4 3.7 17.0<br>53.5 3.7 17.0 | 154-155 | 247 |
| 18 | Ph | Ph | H | 64.8 4.6 17.4<br>65.2 4.7 17.3 | 122-124 | 241 |

Example B1

The fungicidal activity of compounds of the invention was investigated by means of the following tests.

(a) Direct protectant activity against vine downy mildew (Plasmopara viticola; Pvp)

The test is a direct protectant one, using a foliar spray. The lower surfaces of leaves of whole vine plants (cv Cabernet Sauvignon) are sprayed with a solution of active material in 1:1v/v water/acetone containing 0.04%w "Triton X-155" (trade mark) (octylphenol polyoxyethylene surfactant), at a dosage of 1 kilogram of active material per hectare using a track sprayer which delivers 620 litres/ha, and after a subsequent 24 hours under normal glasshouse conditions the lower surfaces of the leaves are inoculated by spraying with an aqueous solution containing $10^4$ zoosporangia/ml. The inoculated plants are kept for 24 hours in a high humidity compartment, 5 days under normal glasshouse conditions and then returned for a further 24 hours to high humidity. Assessment is based on the percentage of leaf area covered by sporulation compared with that on control leaves.

(b) Direct protectant activity against vine grey mould (Botrytis cinerea; Bcp)

The test is a direct protectant one using a foliar spray and is effected as described under (a), with the difference that the leaves are inoculated by spraying with an aqueous solution containing $10^5$ conidia/ml.

(c) Activity against wheat eyespot (Pseudocereosporella herpotrichoides; Ph).

The test is an in vitro one. Samples are prepared wherein 0.7 mls solution containing 2 mg active material dissolved in acetone is evenly dispersed in 20ml molten half-strength potato dextrose agar (formed by dissolving 2g potato extract, 10g dextrose and 7.5g agar in 1 litre of water and sterilising for 15 minutes at 121 °C) and the resulting 20ml portions are allowed to set in 9cm petri dishes. The concentration of active material in the resulting samples is 100ppm. Upon setting, two plugs of 5mm diameter taken from the advancing edge of a stock plate of a 3 to 4 week old culture of P.herpotrichoides on full strength potato dextrose agar, incubated at 20-22 °C in darkness, are placed, equally spaced on the surface of each sample, mycelial side uppermost. The samples are incubated for 11 days at 20-22 °C in darkness before assessment. Diametric growth is measured with the width of the plug subtracted and results compared with growth on a sample wherein 0.7ml acetone containing no active material is dispersed in 20ml half-strength potato agar.

(d) Activity against seedling wheat blight (Fusarium culmorium; Fs)

The test is an anti-sporulant one using a soil drench. Surface sterilised wheat seeds (var Waggoner) are inoculated by soaking in an aqueous suspension containing 7 x $10^5$ spores/ml (60mg seed per 80ml suspension) at 22 °C for 6 hours. The seeds are then sown in pots (5 per pot) in sand at a depth of 1cm. 1 day after inoculation and planting the active material is applied at a rate of 10kg/ha by pouring on a soil drench (concentration 0.36g/l active material in 12%v/v acetone/water) evenly over the sand. The pots are then transferred to glasshouse, kept at 25 °C and watered sparingly. 21 days after inoculation the resulting seedlings are removed from the pots and their roots are gently washed. Visual assessment is made based on lesion development on stem base and upper roots in comparison with control seedlings.

(e) Activity against wheat leafspot (Leptosphaeria nodorum; Ln)

The test is a direct antisporulant one, using a foliar spray. Leaves of wheat plants (cv Mardler), at the single leaf stage, are inoculated by spraying with an aqueous suspension containing 8 x $10^5$ spores/ml. The inoculated plants are kept for 24 hours in a high humidity compartment prior to treatment. The plants are sprayed at a dosage of 1 kg. of active material per hectare using a track sprayer as described under (a). After drying, the plants are kept for 5 days under normal glasshouse conditions, followed by assessment. Assessment is based on the percentage of leaf area covered by sporulation compared with that on leaves of control plants.

(f) Activity against barley powdery mildew (Erysiphe graminis f.sp. hordei; Eg)

The test is a direct antisporulant one, using a foliar spray. Leaves of barley seedlings, cultivar Golden Promise, are inoculated by dusting with mildew conidia one day prior to treatment with the test compound. The inoculated plants are kept overnight at glasshouse ambient temperature and humidity prior to treatment. The plants are sprayed at a dosage of 1kg. of active material per hectare using a track sprayer

as described under (a). After drying, plants are returned to a compartment at ambient temperature and humidity for up to 7 days, followed by assessment. Assessment is based on the percentage of leaf area covered by sporulation compared with that on leaves of control plants.

(g) Activity against tomato early blight (Alternaria Solani;As)

The test is a direct protectant one using a foliar spray. The upper surfaces of leaves of young tomato plants are sprayed with a solution of active material as described in (a) above. After 24 hours under normal glasshouse conditions, the upper surfaces of the leaves are inoculated by spraying with an aqueous suspension containing $10^4$ spores/ml. The inoculated plants are kept for 72 hours in a high humidity compartment and are then removed to lower humidity (50-70% relative humidity). Assessment is made 8 days after inoculation.

(h) Activity against wheat brown rust Puccinia Recondita; Pr)

The test is a direct protectant one using a foliar spray. Wheat seedlings (cv Brigand) are grown to the 1-1.5 leaf stage. The plants are then sprayed with the test compound at a dosage of 1 kg/ha using a track sprayer as described under (a). Test compounds are applied as solutions or suspensions in a mixture of acetone and water (50:50 $^{v}/_{v}$) containing 0.04% surfactant ("TWEEN 20" - Trade Mark).

18-24 hours after treatment, the seedlings are inoculated by spraying the plants from all sides with an aqueous spore suspension containing about $10^5$ spores/ml. For 18 hours after inoculation, the plants are kept in high humidity conditions at a temperature of 20-22°C. Thereafter, the plants are kept in ambient glasshouse conditions, that is, in moderate relative humidity and at a temperature of 20°C.

The disease is assessed 10 days after inoculation on the basis of the percentage of the plant covered by sporulating pustules compared with that on the control plants.

(i) Antisporulant activity against vine downy mildew (Plasmopara viticola; Pva)

The test is a direct antisporulant one using a foliar spray. The lower surfaces of leaves of whole vine plants (cv Cabernet Sauvignon) are inoculated by spraying with an aqueous suspension containing $10^4$ zoosporangia/ml 2 days prior to treatment with the test compound. The inoculated plants are kept for 24 hours in a high humidity compartment, and then 24 hours at glasshouse ambient temperature and humidity. When the plants are dry, infected leaves are sprayed on their lower surfaces with a solution of active material in 1:1 water/acetone containing 0.04% w/w "Triton X-155" (trade mark) (an octylphenol polyethoxylate surfactant). The spraying is carried out with a moving track sprayer with delivers 620 litre/ha, and the concentration of active material is calculated to give an application rate of 1kg/ha. After spraying, the plants are returned to normal glasshouse conditions for 96 hours and are then transferred to the high humidity compartment for 24 hours to induce sporulation, prior to assessment. Assessment is visual and is based on the percentage of the leaf area covered by sporulation compared with that on control leaves.

(j) Activity against rice leaf blast (Pyricularia oryzae;Po)

The test is a direct eradicant one using a foliar spray. The leaves of rice seedlings (about 30 seedlings per pot) are sprayed with an aqueous suspension containing $10^5$ spores/ml 20-24 hours prior to treatment with the test compound. The inoculated plants are kept overnight in high humidity and then allowed to dry before spraying at a dosage of 1kg of active material per hectare using a track sprayer as described under (a). After treatment the plants are kept in a rice compartment at 25-30°C and high humidity. Assessments are made 4-5 days after treatment and are based on the density of necrotic lesions and the degree of withering when compared with control plants.

The extent of disease control in all the above tests is expressed as a rating compared with either an untreated control or a diluent-sprayed-control, according to the criteria:-

0 = less than 50% disease control
1 = about 50-80% disease control
2 = greater than 80% disease control

The results of the above tests are given in Table 3 below.

TABLE 3

| Ex.No. | Pvp | Bcp | Ph | Fs | Ln | Eg | As | Pr | Pva | Po |
|---|---|---|---|---|---|---|---|---|---|---|
| 1 | 2 | | | 2 | 1 | 2 | 2 | 2 | 2 | 2 |
| 2 | 1 | 1 | 2 | 2 | | | | | | |
| 3 | 2 | | 1 | 2 | | 2 | | 1 | 2 | 2 |
| 4 | 1 | | 1 | 2 | 1 | 2 | 2 | 2 | 2 | 2 |
| 5 | | | | 1 | | | | | | |
| 6 | | | | 1 | | | | | | |
| 7 | | | 1 | 1 | 1 | 2 | | 2 | | 1 |
| 8 | | | 2 | 2 | | 2 | 1 | 2 | | 2 |
| 9 | 2 | | 1 | 1 | | 2 | | 2 | | 2 |
| 10 | 1 | | 1 | 1 | | | | | | |
| 11 | | | 1 | 2 | | | | | | |
| 12 | | | 1 | | | | | | 1 | |
| 13 | 1 | | 1 | | | 2 | | 2 | | 1 |
| 14 | | | | 1 | | 2 | | 2 | 2 | 1 |
| 15 | 1 | 2 | 2 | 2 | | 2 | | 2 | 2 | |
| 16 | | | | 1 | | 1 | | 2 | | |
| 17 | | | | 1 | | | | | | |
| 18 | 2 | | 2 | 2 | 1 | 2 | 1 | | | |

The fungicidal activity of certain of the compounds was investigated further, in secondary screening. These revealed that certain compounds were found to have particularly high activity against certain fungi. Example 1 was found to have especially interesting activity against Fs, and Example 9, against Po.

Certain of the compounds, for example Example 3, where shown to have nematicidal activity.

## Claims

### Claims for the following Contracting States : DE, GB, FR, IT, NL, LI, CH, GR

1. A method of combating a fungus at a locus, which method comprises treating the locus with a compound of the general formula

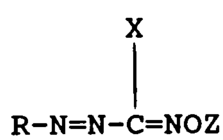

(I)

where R represents an optionally substituted aryl or heteroaryl group; X represents an amino group or an optionally substituted alkyl, aryl or heteroaryl group; and Z represents a hydrogen atom or alkanoyl group.

2. A method as claimed in Claim 1, wherein R represents a phenyl or halogen-substituted phenyl group.

3. A method as claimed in Claim 1 or 2, wherein X represents an optionally substituted alkyl or phenyl group, or an amino, thienyl or pyridyl (including N-oxide) group.

4. A method as claimed in Claim 3 wherein X represents an alkyl or phenyl group.

5. A method as claimed in any preceding Claim, where Z represents a hydrogen atom.

6. Use as a fungicide of a compound of general formula I, as defined in any one of Claims 1 to 5.

7. A compound of general formula I, as claimed in any preceding claim, with the provisos that
   (i) X does not represent an 2,6-dichlorophenyl group;

(ii) when Z represents a hydrogen atom and R represents a phenyl group, then X does not represent a phenyl,2-chlorophenyl, 3-chlorophenyl,4-chlorophenyl, 3-bromophenyl,4-bromophenyl,2-nitrophenyl, 3-nitrophenyl,4-nitrophenyl,4-methylphenyl, 4-methoxyphenyl,3,4-methylenedioxyphenyl,2-furyl,2-thienyl,methyl,n-propyl,benzyl or trifluoromethyl group;

(iii) when Z represents a hydrogen atom and R represents a 4-methylphenyl group, then X does not represent a phenyl,4-methylphenyl, 1-naphthyl,9-anthryl,2-furyl,2-thienyl,2-pyridyl,3-pyridyl or 4-pyridyl group;

(iv) when Z represents a hydrogen atom and R represents a 4-nitrophenyl group, then X does not represent a phenyl group; and

(v) when Z represents a hydrogen atom and X represents an amino group, then R does not represent a phenyl,4-chlorophenyl, 4-bromophenyl,4-nitrophenyl,4-methylphenyl or 4-methoxyphenyl group.

**8.** A process for the preparation of a compound of the general formula I, as claimed in Claim 7, which comprises:

(a) treating a compound of the general formula

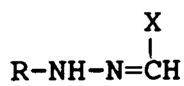

(II)

where R and X are as claimed in Claim 7, but X does not represent an amino group, with a higher alkyl nitrite, in the presence of a base; or

(b) when X represents an amino group, treating a compound of general formula R-$N_2$CN with hydroxylamine or an acid addition salt thereof; or

(c) treating a compound of the general formula

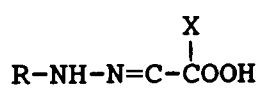

(III)

where R and X are as defined by Claim 7, but X is not an amino group, with a base and a nitrite salt; to yield in each case a compound of the general formula I wherein Z is hydrogen; and optionally derivatising that compound to produce a further novel compound of formula I in which Z represents an alkanoyl group.

**9.** A fungicidal composition which comprises a carrier and, as active ingredient, a compound of general formula I, as claimed in Claim 7.

**Claims for the following Contracting State : ES**

**1.** A method of combating a fungus at a locus, which method comprises treating the locus with a compound of the general formula

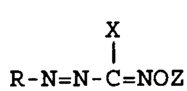

(I)

where R represents an optionally substituted aryl or heteroaryl group; X represents an amino group or an optionally substituted alkyl, aryl or heteroaryl group; and Z represents a hydrogen atom or alkanoyl group.

**2.** A method as claimed in Claim 1, wherein R represents a phenyl or halogen-substituted phenyl group.

**3.** A method as claimed in Claim 1 or 2, wherein X represents an optionally substituted alkyl or phenyl group, or an amino, thienyl or pyridyl (including N-oxide) group.

**4.** A method as claimed in Claim 3 wherein X represents an alkyl or phenyl group.

**5.** A method as claimed in any preceding Claim, where Z represents a hydrogen atom.

**6.** Use as a fungicide of a compound of general formula I, as defined in any one of Claims 1 to 6.

**7.** A method as claimed in any one of Claims 1 to 5, with the provisos that
(i) X does not represent a 2,6-dichlorophenyl group;
(ii) when Z represents a hydrogen atom and R represents a phenyl group, then X does not represent a phenyl,2-chlorophenyl, 3-chlorophenyl,4-chlorophenyl, 3-bromophenyl, 4-bromophenyl, 2-nitrophenyl, 3-nitrophenyl, 4-nitrophenyl, 4-methylphenyl, 4-methoxyphenyl,3,4-methylenedioxyphenyl, 2-furyl,2-thienyl, methyl, n-propyl, benzyl or trifluoromethyl group;
(iii) when Z represents a hydrogen atom and R represents a 4-methylphenyl group, then X does not represent a phenyl, 4-methylphenyl, 1-naphthyl, 9-anthryl, 2-furyl, 2-thienyl, 2-pyridyl, 3-pyridyl or 4-pyridyl group;
(iv) when Z represents a hydrogen atom and R represents a 4-nitrophenyl group, then X does not represent a phenyl group; and
(v) when Z represents a hydrogen atom and X represents an amino group, then R does not represent a phenyl, 4-chlorophenyl, 4-bromophenyl, 4-nitrophenyl, 4-methylphenyl or 4-methoxyphenyl group.

**8.** A process for the preparation of a compound of the general formula I, as defined in Claim 7, which comprises:
(a) treating a compound of the general formula

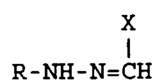

(II)

where R and X are as claimed in Claim 7, but X does not represent an amino group, with a higher alkyl nitrite, in the presence of a base; or
(b) when X represents an amino group, treating a compound of general formula $R\text{-}N_2CN$ with hydroxylamine or an acid addition salt thereof; or
(c) treating a compound of the general formula

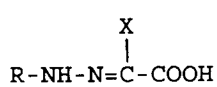

(III)

wherein R and X are as defined by Claim 7, but X is not an amino group, with a base and a nitrite salt;
to yield in each case a compound of the general formula I wherein Z is hydrogen; and optionally derivatising that compound to produce a further novel compound of formula I in which Z represents an alkanoyl group.

**9.** A fungicidal composition which comprises a carrier and, as active ingredient, a compound of general formula I, as defined in Claim 7.

**Patentansprüche**
**Patentansprüche für folgende Vertragsstaaten : DE, GB, FR, IT, NL, LI, CH, GR**

**1.** Ein verfahren zum Bekämpfen eines Fungus an einem Ort, welches Verfahren ein Behandeln des Ortes mit einer Verbindung der allgemeinen Formel

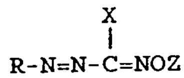

(I)

umfaßt, in der R eine gegebenenfalls substituierte Aryl- oder Heteroarylgruppe darstellt; X eine Amino-Gruppe oder eine gegebenenfalls substituierte Alkyl-, Aryl- oder Heteroaryl-Gruppe darstellt; und Z ein Wasserstoffatom oder eine Alkanoylgruppe darstellt.

**2.** Ein Verfahren wie in Anspruch 1 beansprucht, wobei R eine Phenyl- oder halogensubstituierte Phenylgruppe darstellt.

**3.** Ein Verfahren wie in Anspruch 1 oder 2 beansprucht, wobei X eine gegebenenfalls substituierte Alkyl- oder Phenylgruppe oder eine Amino-, Thienyl- oder Pyridylgruppe (einschließlich N-Oxid) darstellt.

**4.** Ein Verfahren wie in Anspruch 3 beansprucht, wobei X eine Alkyl- oder Phenylgruppe darstellt.

**5.** Ein Verfahren wie in einem der vorhergehenden Ansprüche beansprucht, wobei Z ein Wasserstoffatom darstellt.

**6.** Verwendung einer Verbindung der allgemeinen Formel I, wie in irgendeinem der Ansprüche 1 - 5 definiert, als ein Fungizid.

**7.** Eine Verbindung der allgemeinen Formel I, wie in irgendeinem der vorhergehenden Ansprüche beansprucht, mit der Maßgabe, daß
(i) X keine 2,6-Dichlorphenyl-Gruppe darstellt;
(ii) wenn Z ein Wasserstoffatom darstellt und R eine Phenyl-Gruppe darstellt, dann X keine Phenyl-, 2-Chlorphenyl-, 3-Chlorphenyl-, 4-Chlorphenyl-, 3-Bromphenyl-, 4-Bromphenyl-, 2-Nitrophenyl-, 3-Nitrophenyl-, 4-Nitrophenyl-, 4-Methylphenyl-, 4-Methoxyphenyl-, 3,4-Methylendioxyphenyl-, 2-Furyl-, 2-Thienyl-, Methyl-, n-Propyl-, Benzyl- oder Trifluormethyl-Gruppe darstellt;
(iii) wenn Z ein Wasserstoffatom darstellt und R eine 4-Methylphenyl-Gruppe darstellt, dann X keine Phenyl-, 4-Methylphenyl-, 1-Naphthyl-, 9-Anthryl-2-Furyl-, 2-Thienyl-, 2-Pyridyl-, 3-Pyridyl- oder 4-Pyridyl-Gruppe darstellt;
(iv) wenn Z ein Wasserstoffatom darstellt und R eine 4-Nitrophenyl-Gruppe darstellt, dann X keine Phenylgruppe darstellt; und
(v) wenn Z ein Wasserstoffatom darstellt und X eine Amino-Gruppe darstellt, dann R keine Phenyl-, 4-Chlorphenyl-, 4-Bromphenyl-, 4-Nitrophenyl-, 4-Methylphenyl- oder 4-Methoxyphenyl-Gruppe darstellt.

**8.** Ein Verfahren zur Herstellung einer Verbindung der allgemeinen Formel I, wie in Anspruch 7 beansprucht, das umfaßt
(a) Behandeln einer Verbindung der allgemeinen Formel

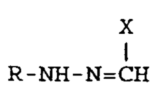

(II)

in der R und X wie in Anspruch 7 beansprucht sind, jedoch X keine Aminogruppe darstellt, mit einem höheren Alkylnitrit in Gegenwart einer Base; oder
(b) wenn X eine Aminogruppe darstellt, Behandeln einer Verbindung der allgemeinen Formel $R-N_2CN$ mit Hydroxylamin oder dessen Säureadditionssalz; oder
(c) Behandeln einer Verbindung der allgemeinen Formel

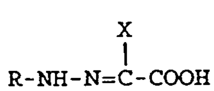

(III)

wobei R und X wie durch Anspruch 7 definiert sind, jedoch X keine Aminogruppe ist, mit einer Base und einem Nitritsalz; zur Lieferung einer Verbindung der allgemeinen Formel I in jedem Falle, in der Z Wasserstoff ist, und gegebenenfalls Derivatisierung jener Verbindung zur Erzeugung einer weiteren neuen Verbindung der Formel I, in der Z eine Alkanoyl-Gruppe darstellt.

**9.** Eine fungizide Zusammensetzung, die ein Trägermaterial und - als aktiven Bestandteil - eine Verbindung der allgemeinen Formel I, wie in Anspruch 7 beansprucht, umfaßt.

**Patentansprüche für folgenden Vertragsstaat : ES**

**1.** Ein Verfahren zum Bekämpfen eines Fungus an einem Ort, welches Verfahren ein Behandeln des Ortes mit einer Verbindung der allgemeinen Formel

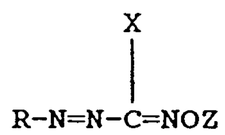

(I)

umfaßt, in der R eine gegebenenfalls substituierte Aryl- oder Heteroarylgruppe darstellt; X eine Amino-Gruppe oder eine gegebenenfalls substituierte Alkyl-, Aryl- oder Heteroaryl-Gruppe darstellt; und Z ein Wasserstoffatom oder eine Alkanoylgruppe darstellt.

**2.** Ein Verfahren wie in Anspruch 1 beansprucht, wobei R eine Phenyl- oder halogensubstituierte Phenylgruppe darstellt.

**3.** Ein Verfahren wie in Anspruch 1 oder 2 beansprucht, wobei X eine gegebenenfalls substituierte Alkyl- oder Phenylgruppe oder eine Amino-, Thienyl- oder Pyridylgruppe (einschließlich N-Oxid) darstellt.

**4.** Ein Verfahren wie in Anspruch 3 beansprucht, wobei X eine Alkyl- oder Phenylgruppe darstellt.

**5.** Ein Verfahren wie in einem der vorhergehenden Ansprüche beansprucht, wobei Z ein Wasserstoffatom darstellt.

**6.** Verwendung einer Verbindung der allgemeinen Formel I, wie in irgendeinem der Ansprüche 1 - 5 definiert, als ein Fungizid.

**7.** Ein Verfahren, wie in irgendeinem der Ansprüche 1 bis 5 beansprucht, mit der Maßgabe, daß
(i) X keine 2,6-Dichlorphenyl-Gruppe darstellt;
(ii) wenn Z ein Wasserstoffatom darstellt und R eine Phenyl-Gruppe darstellt, dann X keine Phenyl-, 2-Chlorphenyl-, 3-Chlorphenyl-, 4-Chlorphenyl-, 3-Bromphenyl-, 4-Bromphenyl-, 2-Nitrophenyl-, 3-Nitrophenyl-, 4-Nitrophenyl-, 4-Methylphenyl-, 4-Methoxvphenyl-, 3,4-Methylendioxyphenyl-, 2-Furyl-, 2-Thienyl-, Methyl-, n-Propyl-, Benzyl- oder Trifluormethyl-Gruppe darstellt;
(iii) wenn Z ein Wasserstoffatom darstellt und R eine 4-Methylphenyl-Gruppe darstellt, dann X keine Phenyl-, 4-Methylphenyl-, 1-Naphthyl-, 9-Anthryl-2-Furyl-, 2-Thienyl-, 2-Pyridyl-, 3-Pyridyl- oder 4-Pyridyl-Gruppe darstellt;
(iv) wenn Z ein Wasserstoffatom darstellt und R eine 4-Nitrophenyl-Gruppe darstellt, dann X keine Phenylgruppe darstellt; und
(v) wenn Z ein Wasserstoffatom darstellt und X eine Amino-Gruppe darstellt, dann R keine Phenyl-, 4-Chlorphenyl-, 4-Bromphenyl-, 4-Nitrophenyl-, 4-Methylphenyl- oder 4-Methoxyphenyl-Gruppe darstellt.

**8.** Ein Verfahren zur Herstellung einer Verbindung der allgemeinen Formel I, wie in Anspruch 7 definiert, das umfaßt

(a) Behandeln einer Verbindung der allgemeinen Formel

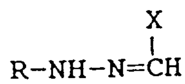

(II)

in der R und X wie in Anspruch 7 beansprucht sind, jedoch X keine Aminogruppe darstellt, mit einem höheren Alkylnitrit in Gegenwart einer Base; oder
(b) wenn X eine Aminogruppe darstellt, Behandeln einer Verbindung der allgemeinen Formel R-$N_2CN$ mit Hydroxylamin oder dessen Säureadditionssalz; oder
(c) Behandeln einer Verbindung der allgemeinen Formel

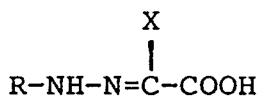

(III)

wobei R und X wie durch Anspruch 7 definiert sind, jedoch X keine Aminogruppe ist, mit einer Base und einem Nitritsalz; zur Lieferung einer Verbindung der allgemeinen Formel I in jedem Falle, in der Z Wasserstoff ist, und gegebenenfalls Derivatisierung jener Verbindung zur Erzeugung einer weiteren neuen Verbindung der Formel I, in der Z eine Alkanoyl-Gruppe darstellt.

**9.** Eine fungizide Zusammensetzung, die ein Trägermaterial und - als aktiven Bestandteil - eine Verbindung der allgemeinen Formel I, wie in Anspruch 7 definiert, umfaßt.

**Revendications**

**Revendications pour les Etats contractants suivants : DE, GB, FR, IT, NL, LI, CH, GR**

**1.** Une méthode de lutte contre un champignon en un lieu, qui comprend le traitement du lieu avec un composé de la formule générale

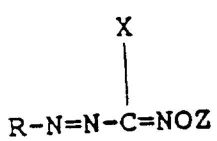

(I)

dans laquelle R représente un groupe aryle ou hétéro-aryle éventuellement substitué ; X représente un groupe amino ou un groupe alcoyle, aryle ou hétéro-aryle éventuellement substitué ; et Z représente un atome d'hydrogène ou un groupe alcanoyle.

**2.** Une méthode selon la revendication 1, dans laquelle R représente un groupe phényle ou un groupe phényle halogéné.

**3.** Une méthode selon la revendication 1 ou 2, dans laquelle X représente un groupe alcoyle ou phényle éventuellement substitué ou un groupe amino, thiényle ou pyridyle (y compris le N-oxyde).

**4.** Une méthode selon la revendication 3, dans laquelle X représente un groupe alcoyle ou phényle.

**5.** Une méthode selon l'une quelconque des revendications précédentes, dans laquelle Z représente un atome d'hydrogène.

**6.** Utilisation comme fongicide d'un composé de formule générale I, tel que défini dans l'une quelconque des revendications 1 à 5.

**7.** Un composé de formule générale I, selon l'une quelconque des revendications précédentes, avec les conditions que

(1) X ne représente pas un groupe 2,6-dichlorophényle ;

(2) quand Z représente un atome d'hydrogène et R représente un groupe phényle, alors X ne représente pas un groupe phényle, 2-chlorophényle, 3-chlorophényle, 4-chlorophényle, 3-bromophényle, 4-bromophényle, 2-nitrophényle, 3-nitrophényle, 4-nitrophényle, 4-méthylphényle, 4-méthoxyphényle, 3,4-méthylènedioxyphényle, 2-furyle, 2-thiényle, méthyle, n-propyle, benzyle ou trifluorométhyle ;

(3) quand Z représente un atome d'hydrogène et R représente un groupe 4-méthylphényle, alors X ne représente pas un groupe phényle, 4-méthylphényle, 1-naphtyle, 9-anthryle, 2-furyle, 2-thiényle, 2-pyridyle, 3-pyridyle ou 4-pyridyle ;

(4) quand Z représente un atome d'hydrogène et R représente un groupe 4-nitrophényle, alors X ne représente pas un groupe phényle ; et

(5) quand Z représente un atome d'hydrogène et X représente un groupe amino, alors R ne représente pas un groupe phényle, 4-chlorophényle, 4-bromophényle, 4-nitrophényle, 4-méthylphényle ou 4-méthoxyphényle.

**8.** Un procédé pour la préparation d'un composé de la formule générale I, tel que revendiqué dans la revendication 7, qui comprend :

(a) le traitement d'un composé de la formule générale

$$R\text{-}NH\text{-}N=\overset{X}{\overset{|}{C}}H \qquad (II)$$

dans laquelle R et X sont tels que définis dans la revendication 7, mais X ne représente pas un groupe amino, avec un nitrite d'alcoyle supérieur, en présence d'une base ; ou

(b) quand X représente un groupe amino, le traitement d'un composé de formule générale $R\text{-}N_2CN$ avec l'hydroxylamine ou un sel d'addition d'acide de ce composé ; ou

(c) le traitement d'un composé de la formule générale

$$R\text{-}NH\text{-}N=C\text{-}\overset{X}{\overset{|}{C}}OOH \qquad (III)$$

dans laquelle R et X sont tels que définis par la revendication 7, mais X n'est pas un groupe amino, avec une base et un sel nitrite ;

pour donner dans chaque cas un composé de la formule générale I dans lequel Z est l'hydrogène ; et éventuellement la transformation de ce composé en un dérivé pour produire un autre composé nouveau de formule I dans lequel Z représente un groupe alcanoyle.

**9.** Une composition fongicide qui comprend un véhicule et, comme ingrédient actif, un composé de formule générale I, tel que revendiqué dans la revendication 7.

**Revendications pour l'Etat contractant suivant : ES**

**1.** Une méthode de lutte contre un champignon en un lieu, qui comprend le traitement du lieu avec un composé de la formule générale

$$R\text{-}N=N\text{-}\overset{X}{\overset{|}{C}}=NOZ \qquad (I)$$

dans laquelle R représente un groupe aryle ou hétéro-aryle éventuellement substitué ; X représente un groupe amino ou un groupe alcoyle, aryle ou hétéro-aryle éventuellement substitué ; et Z représente un atome d'hydrogène ou un groupe alcanoyle.

**2.** Une méthode selon la revendication 1, dans laquelle R représente un groupe phényle ou un groupe phényle halogéné.

**3.** Une méthode selon la revendication 1 ou 2, dans laquelle X représente un groupe alcoyle ou phényle éventuellement substitué ou un groupe amino, thiényle ou pyridyle (y compris le N-oxyde).

**4.** Une méthode selon la revendication 3, dans laquelle X représente un groupe alcoyle ou phényle.

**5.** Une méthode selon l'une quelconque des revendications précédentes, dans laquelle Z représente un atome d'hydrogène.

**6.** Utilisation comme fongicide d'un composé de formule générale I, tel que défini dans l'une quelconque des revendications 1 à 5.

**7.** Une méthode selon l'une quelconque des revendications 1 à 5, avec les conditions que
(1) X ne représente pas un groupe 2,6-dichlorophényle ;
(2) quand Z représente un atome d'hydrogène et R représente un groupe phényle, alors X ne représente pas un groupe phényle, 2-chlorophényle, 3-chlorophényle, 4-chlorophényle, 3-bromophényle, 4-bromophényle, 2-nitrophényle, 3-nitrophényle, 4-nitrophényle, 4-méthylphényle, 4-méthoxyphényle, 3,4-méthylènedioxyphényle, 2-furyle, 2-thiényle, méthyle, n-propyle, benzyle ou trifluorométhyle ;
(3) quand Z représente un atome d'hydrogène et R représente un groupe 4-méthylphényle, alors X ne représente pas un groupe phényle, 4-méthylphényle, 1-naphtyle, 9-anthryle, 2-furyle, 2-thiényle, 2-pyridyle, 3-pyridyle ou 4-pyridyle ;
(4) quand Z représente un atome d'hydrogène et R représente un groupe 4-nitrophényle, alors X ne représente pas un groupe phényle ; et
(5) quand Z représente un atome d'hydrogène et X représente un groupe amino, alors R ne représente pas un groupe phényle, 4-chlorophényle, 4-bromophényle, 4-nitrophényle, 4-méthylphényle ou 4-méthoxyphényle.

**8.** Un procédé pour la préparation d'un composé de la formule générale I, tel que défini dans la revendication 7, qui comprend :
(a) le traitement d'un composé de la formule générale

$$R\text{-}NH\text{-}N=\overset{X}{C}H \qquad (II)$$

dans laquelle R et X sont tels que définis dans la revendication 7, mais X ne représente pas un groupe amino, avec un nitrite d'alcoyle supérieur, en présence d'une base ; ou
(b) quand X représente un groupe amino, le traitement d'un composé de formule générale $R\text{-}N_2CN$ avec l'hydroxylamine ou un sel d'addition d'acide de ce composé ; ou
(c) le traitement d'un composé de la formule générale

$$R\text{-}NH\text{-}N=\overset{X}{C}\text{-}COOH \qquad (III)$$

dans laquelle R et X sont tels que définis par la revendication 7, mais X n'est pas un groupe amino, avec une base et un sel nitrite ;
pour donner dans chaque cas un composé de la formule générale I dans lequel Z est l'hydrogène ; et éventuellement la transformation de ce composé en un dérivé pour produire un autre composé nouveau de formule I dans lequel Z représente un groupe alcanoyle.

**9.** Une composition fongicide qui comprend un véhicule et, comme ingrédient actif, un composé de formule générale I, tel que défini dans la revendication 7.